# EUROPÄISCHE PATENTSCHRIFT

(11) **EP 2 200 542 B1**
(45) Veröffentlichungstag und Bekanntmachung des Hinweises auf die Patenterteilung: **30.04.2014**
(21) Anmeldenummer: 08841863.7
(22) Anmeldetag: 30.09.2008
(51) Int. Cl.: A61F 2/46

(54) **UNIVERSALAUSSCHLÄGER FÜR TOTALENDOPROTHESEN DES HÜFTGELENKES, LANGSCHAFTKOMPONENTEN MODULARER PROTHESENSYSTEME ODER MARKNÄGEL**
UNIVERSAL EXTRACTOR FOR TOTAL ENDOPROSTHESES OF THE HIP JOINT, MODULAR ELONGATE SHAFT COMPONENTS OF PROSTHETIC SYSTEMS OR INTRAMEDULLARY PINS
EXTRACTEUR UNIVERSEL POUR PROTHÈSES TOTALES DE L'ARTICULATION DE LA HANCHE, ÉLÉMENTS À TIGE ALLONGÉE DE SYSTÈMES DE PROTHÈSE MODULAIRES OU CLOUS INTRAMÉDULLAIRES

(30) Priorität: 23.10.2007 DE 102007051293
(43) Veröffentlichungstag der Anmeldung: 30.06.2010
(73) Patentinhaber: Arnhold, Christian, 99817 Eisenach (DE)
(72) Erfinder: ARNHOLD, Michael, 07607 Eisenberg (DE)
(74) Vertreter: Donath, Dirk
(86) Internationale Anmeldenummer: PCT/DE2008/001611
(87) Internationale Veröffentlichungsnummer: WO 2009/052776

(56) Entgegenhaltungen:
- EP-A- 0 743 049
- FR-A- 2 749 501

## Beschreibung

Die Erfindung betrifft einen Universalausschläger für Hüftendoprothesen, Langschaftkomponenten modularer Prothesensysteme oder Marknägel gemäß der Gattung der Patentansprüche.

Verschiedenste Bauformen von chirurgischen Spannvorrichtungen und Ausschlägern sind seit Jahren bekannt. Insbesondere ist die Wirkungsweise der Ausschläger oft unbefriedigend.

Die Totalendoprothesenplastik (TEP) des Hüftgelenkes ist die am häufigsten durchgeführte Gelenkersatzoperation. Dabei wird das verschlissene natürliche Gelenk durch ein technisches Gelenk ersetzt, welches jedoch sofort einem Verschleiß unterliegt. Auch Infektionen des Implantates sind zwar selten, aber nicht ausgeschlossen.

Die Folge ist eine steigende Zahl von Wechseloperationen, die als ersten Scluitt eine das knöcherne Implantatlager schonende Entfernung der Prothesenteile notwendig macht, bevor eine Reimplantation einer neuen Prothese möglich wird. Bei septischen Wechseloperationen erfolgt in der Regel ein zweiseitiges Vorgehen mit temporärer Implantation eines Spacers aus Knochenzement.

Bis vor 20 Jahren war der Markt von zementierten Implantaten beherrscht, d.h. die Schaft- und Pfannenkomponenten wurden mit Knochenzement im natürlichen - Knochen verankert. Die knochenschonende Entfernung derartiger Prothesen war einfach, da die Schaftkomponenten meist einen Kragen besaßen, der einen günstigen Angriffspunkt zum Ausschlagen darstellte.

In der Folgezeit haben kragenlose, zementfreie und modulare Prothesensysteme bis zum heutigen Tag einen hohen Marktanteil erobert. Die Entfernung dieser ist jedoch ungleich problematischer; insbesondere wenn die knöcherne Integration bei langen zementfreien Prothesenschäften großflächige bis weit in die distalen Markraumabschnitte erfolgt ist. Weiterhin erschwerend für den handelnden Operateur erweist sich die breite Palette unterschiedlicher Prothesentypen zahlreicher Hersteller.

Nur wenige Prothesenproduzenten haben sich der Demontage bzw. Entfernung der Schaftkomponenten gestellt. Bei der überwiegenden Mehrheit kommen gemäß dem Stand der Technik Universalausschläger zur Anwendung, die als Verankerungspunkt den Schaftkonus, der das einzige genormte Teil des Schaftes darstellt, nutzen. Die Beanspruchung eines Konus auf Zug ist jedoch mechanisch äußerst unzweckmäßig. Oft weisen die Halssegmente der Prothesen unterhalb des Konus eine ungenügende Verjüngung auf, die eine stabile Verankerung des Ausschlägers oft unmöglich macht. Moderne Prothesen sind zudem meist kragenlos, so dass ein tangential zur Schaftachse wirkendes Ausschlaginstrument keinen Angriffspunkt findet. Derartige Hilfsmittel sind wegen ihrer auf das knöcherne Lager wirkenden Scherkräfte problematisch. Ungewollte Schaftfrakturen oder zusätzliche Knochendefekte können die Folge sein. Ebenso unzweckmäßig sind die bisher häufig gebräuchlichen Sledge-Hammer-Vorrichtungen am Ausschläger, die meist nur eine ungenügende Kraftentfaltung ermöglichen und z.T. durch ihre überdimensionierten Ausmaße dem Operateur die Arbeit erschweren.

Bleiben alle Bemühungen des Operateurs bei der Explantation eines fest knöchern integrierten Prothesenschaftes erfolglos, so bleibt ihm als Ultima ratio nur die langstreckige Osteotomie des knöchernen Prothesenlagers. Damit geht wertvoller knöcherner Markraum für die Reimplantation eines Prothesenschaftes verloren.

Deutlich problematischer wird dieser Umstand, wenn die Explantation einer festen zementfreien modularen Langschaftprothese oder eines fest verankerten Marknagels nach Osteosynthese bei fehlendem Originalinstrumentarium erfolgen muss. In diesen Fällen sind bereits die Reserven von intakten, knöchernen Markraum für eine Reimplantation nahezu ausgeschöpft. Um ein risikoreiches Großimplantat mit schlechter Gelenkfunktion und erhöhtem Infektionsrisiko zu vermeiden, muss der Operateur besonders sorgsam mit den "Markraumresten" bei der "Vorlockerung" des langen Implantates vorgehen. Als Angriffspunkt für ein Explantationsinstrument steht dann meist nur ein im "Langimplantat" bestehendes Gewinde unbekannter Größe zur Verfügung. Nach dem Stand der Technik ist ein effektives Entfernungsinstrument nur bei sehr wenigen Prothesen oder Marknägeltypen verfügbar.

Die Schrift DE 100 14 401 A1 offenbart eine Vorrichtung zum Lösen von Konus-Steckverbindungen. Letztere sind bei modularen Hüftprothesen zwischen Schaft und Halssegment oder Gelenkkugel und Prothesenhals anzutreffen. Dieses Instrument weist 2 Schenkel auf, die sich jeweils an den zu lösenden Prothesenteilen abstützen und diese durch eine Spreizbewegung auseinander treiben.

Ein Instrument zum Spannen und Fixieren von Drahtschlingen, sogenannten Cerclagen, um einen Röhrenknochen beschreibt die Schrift DE 296 12 072 U1. Beim Spannvorgang wird dabei das Spannmittel Draht durch einen bewegbaren Schieber axial gezogen und durch ein fixierendes Element gehalten. Die fest am Röhrenknochen haftende Cerclage wird dann verplombt und dient zur Fixierung von Knochenfragmenten bei Frakturen oder Knochentransplantaten.

Aus FR 2 749 501 A1 ist ein Ausschläger für Hüftendoprothesen bekannt, welcher zum Festhalten von Hüftprothesen dient. Dieser Ausschläger ist aus einem T-Stück gebildet, bestehend aus einer Platte und einem Schenkel, wobei das distale Ende des Ausschlägers mit einer Schlaufe mechanisch fest verbunden ist, die an dem der Platte gegenüberliegenden Ende des Ausschlägers heraus ragt. Der Schenkel des Ausschlägers weist einen Gewindestab mit Schraube und Drehgriff auf, um eine Drehbewegung in eine axiale Bewegung der Schlaufe umzuwandeln.

Der Nachteil dieser technischen Lösung besteht darin, dass das distale Ende des Ausschlägers mit der Schlaufe mechanisch fest verbunden ist, so dass die Schlaufe als Verschleißteil nicht auswechselbar ist bzw. dass je nach Bedarf nicht verschiedenste Schlaufen unterschiedlicher Längen beim Einsatz des Ausschlägers nutzbar sind.

Aus US 2001/0051830 A1 ist ein chirurgisches Werkzeug zum Greifen einer Prothese während eines chirurgischen Eingriffs (Fixation von Hüftpfannen bei Implantationen) bekannt, wobei dieses Werkzeug einen Schenkel als axiales Rohr, einen Schraubbolzen, einen Führungsbolzen und eine nicht öffenbare / nicht lösbare Schlaufe aufweist. Dabei erfolgt die Befestigung der Drahtschlaufe über eine mobile Druckplatte vermittels mehrerer Ausnehmungen, durch welche die Drahtschlaufe gehaltert wird.

Der Nachteil dieser technischen Lösung besteht darin, dass Werkzeug nicht bei Explantationen einsetzbar ist und die beiden Enden der durch die Löcher der Druckplatte geführten Schlaufe fest miteinander verbunden sind, so dass die Schlaufe als Verschleißteil nicht auswechselbar ist bzw. dass je nach Bedarf nicht verschiedenste Schlaufen unterschiedlicher Längen beim Einsatz des Ausschlägers nutzbar sind.

Hinzu kommt, dass das Werkzeug nur bei Implantaten einsetzbar ist, welche Verankerungsmöglichkeiten für die Schlaufen aufweisen, da ansonsten kein Anschlingen des Werkzeugs an einem Implantat möglich ist.

Weiterhin ist die Verwendung von Schlaufen (Kordel, Kabel) in Form von Achtertouren in der Chirurgie aus DE 38 10 465 A1 und US 2004/0243131 A1 bekannt, wobei die Achtertouren das Schultergelenk gegen seitliches Auseinanderweichen oder zum Zusammenschnüren von Knochenteilen vermittels der Kordel bzw. Kabel dient.

Der Erfindung liegt daher die Aufgabe zu Grunde, einen Prothesenausschläger für Hüftprothesen, Langschaftkomponenten modularer Prothesensysteme oder Marknägel anzugeben, der die Nachteile des Standes der Technik vermeidet, insbesondere in ihrer Länge einstellbare und mechanisch stabile Anschlingungen des Prothesenschaftes durch den Ausschläger, einen Wechsel der verschlissenen Schlaufen und eine leichte Explantation der Prothesen bei Schonung des knöchernen Lagers ermöglicht.

Voraussetzung für eine erfolgreiche Entfernung ist eine entsprechende "Vorlockerung" des Prothesenschaftes im knöchernen Lager. Das sollte bei zementfreien, knöchern integrierten Schäften mit großer Vorsicht erfolgen. Als Hilfsmittel würden Spezialmeißel und Kirschnerdrähte zur Anwendung kommen. Ziel ist eine Verringerung der knöchernen Integrationsfläche, um die Ausschlagkraft zur Lösung des Implant-Knochen-Verbundes zu minimieren, bei schlechter mechanischer Knochenqualität Sekundärschäden oder nachteilige Osteotomien des Markraumes zu vermeiden.

Dazu müssen u.a. folgende Aufgaben bzgl. des Universalausschlägers gelöst werden:
- eine stabile, orthograde Verankerung an Prothesenschäften unterschiedlichster Designs,
- eine optimale Entfaltung der Zugkraft,
- eine Wiederverwendbarkeit sowie
- eine universelle Anwendung bei Schulter- und Ellenbogenendoprothesen und in einer zweiten speziellen Designform auch bei Langschaftkomponenten modularer Systeme sowie diversen Marknägeln.

Gemäß der Erfindung wird diese Aufgabe durch die kennzeichnenden Merkmale des ersten Patentanspruches gelöst und durch vorteilhafte Ausgestaltungen gemäß den Unteransprüchen ergänzt.

Das Wesen der Erfindung besteht darin, dass der Universalausschläger aus folgenden Teilen aufgebaut ist:
- T-Stück, wobei der axiale Schenkel aus einem Rohr besteht
- Führungsbolzen mit Ausnehmungen zur Aufnahme der freien Enden einer Schlaufe
- Schraubbolzen mit Kurbel oder Sterngriff

Der Führungsbolzen ist im Rohr des T-Stückes axial gelagert und kann eine axiale Bewegung ausführen. Durch eine radial angeordnete Schraube sowie das Langloch im Rohr wird der Freiheitsgrad der Rotation des Führungsbolzens aufgehoben.

Die axiale Verschiebung des Führungsbolzens erfolgt über den axial angeordneten Schraubbolzen. Dieser stützt sich auf der Platte des T-Stückes ab. Der untere Teil des Schraubbolzens ist durch ein Gewinde mit dem Führungsbolzen gekoppeit.

Das Schraubengetriebe bewirkt eine axial auf den Führungsbolzen wirkende Zugkraft, so dass dieser in das Rohr des T-Stückes gezogen wird. Der Führungsbolzen ist mit einer Drahtschlaufe verbindbar, die am unteren Teil des Prothesenausschlägers herausragt.

Die stabile orthograde Verankerung des Ausschlägers erfolgt am Prothesenschaft durch eine Schlaufe, besonders vorteilhaft bestehend aus geflochtenem Material, wie bspw. Metall, welche im Betriebszustand in Form einer Achtertour um dessen Halssegment gelegt wird. Durch den oben beschriebenen Mechanismus wird das Seil der Schlaufe gespannt, wobei der untere Teil des Ausschlägers gegen das Halssegment des Prothesenschaftes gepresst wird. Durch die Form der Kontaktfläche des Ausschlägers erfolgt dessen schrittweise Selbstzentrierung. Nun kann durch Schläge eines Metallhammers gegen die Ausschlagplatte des T-Stückes der "vorgelockerte" Prothesenschaft aus dem knöchernen Markraum gezogen werden. Im Gegensatz zu den bisher üblichen Sledge-Hämmein kann sich die Ausschlagkraft bei dem erfindungsgemäßen Ausschläger optimal entfalten.

Erfindungswesentlich dabei ist, dass die Schlaufe zwei freie Enden besitzt, die mit Halteelementen versehen sind, und dass der distale Schenkel zwei Ausnehmungen zum Einführen der Halteelemente aufweist, denen räumlich korrespondierend Ausnehmungen in dem Führungsbolzen zugeordnet sind, wobei durch die Ausnehmungen die Halteelemente einhängbar und kraftschlüssig halterbar sind.

Durch die einhängbaren Halteelemente, die sich an den freien Enden der Drahtschlaufe befinden ist, diese beliebig austauschbar.

Die Wiederverwendbarkeit des Universalausschlägers ist durch die Reversibilität der Verankerung, die Resterilisierbarkeit und die Ersetzbarkeit des Verschleißteils Schlaufe gesichert, so dass Mehrfachanwendungen des erfindungsgemäßen Ausschlägers problemlos möglich sind.

Die universelle Anwendung des Ausschlägers bei Prothesenschäften unterschiedlichen Designs wird durch die Verankerung mittels Halteelemente-tragender Drahtschlaufe gewährleistet. Eine Anwendung des erfindungsgemäßen Ausschlägers bei Schulter- und Ellenbogenendoprothesen ist ebenfalls möglich möglich.

Die Erfindung wird nachstehend an Hand der schematischen Zeichnungen näher erläutert. Es zeigt:
- Fig. 1:: eine Ausführungsform des erfindungsgemäßen Universalausschlägers in schematischer 3-D-Darstellung,
- Fig. 2:: die Ausführungsform gemäß Fig. 1 in einer Darstellung der auseinander gefügten Einzelteile,
- Fig. 3:: die Ausführungsform gemäß Fig. 1 vor dem Einführen der Schlaufe in schematischer 3-D-Darstellung,
- Fig. 4:: eine Darstellung der Schlaufe und
- Fig. 5:: die Ausführungsform gemäß Fig. 1 in teilweiser Schnittdarstellung im Bereich der eingeführten Schlaufe,

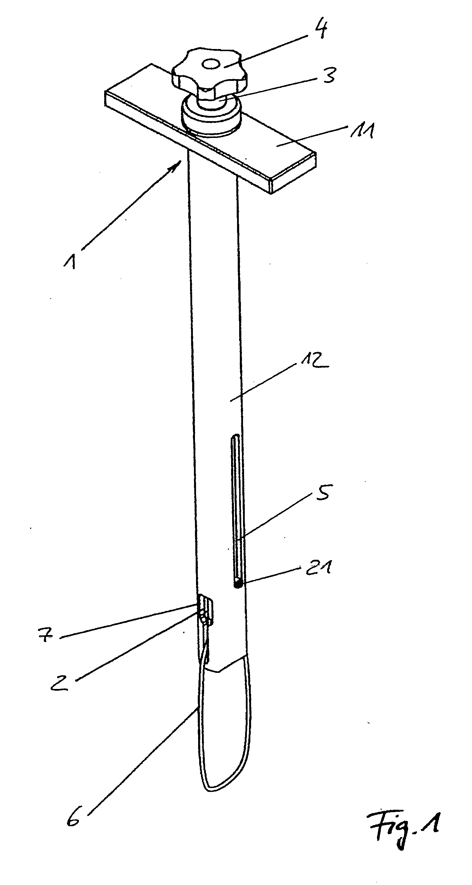

Der in Fig. 1, 2 und 3 dargestellte Universalausschläger besteht aus einem T-Stück 1, welches von der Ausschlagplatte 11 und einem Rohr als axialen Schenkel 12 gebildet wird, einem Führungsbolzen 2 mit Schraube 21 sowie einem Schraubbolzen 3 mit Kurbel oder Sterngriff 4.

Der Führungsbolzen 2 ist im Rohr 12 des T-Stückes 1 axial gelagert und kann eine axiale Bewegung ausführen. Durch eine radial angeordnete Schraube 21, die in einem Langloch 5 des Rohres 12 gleitet, wird der Freiheitsgrad der Rotation des Führungsbolzens 2 aufgehoben.

Die axiale Verschiebung des Führungsbolzens 2 erfolgt über den axial angeordneten Schraubbolzen 3, welcher sich an der Ausschlagplatte 11 des T-Stückes 1 abstützt. Der untere Teil des Schraubbolzens 3 ist durch ein Schraubengetriebe mit dem Führungsbolzen 2 gekoppelt. Das Schraubengetriebe bewirkt in betriebenem Zustand eine axial auf den Führungsbolzen 2 wirkende Zugkraft, so dass dieser in das Rohr 12 des T-Stückes 1 gezogen wird. Der Führungsbolzen 2 ist an seinem distalen Ende mit einer Schlaufe 6, vorteilhaft einer Drahtschlaufe, verbunden, die am distalen Teil des Universalausschlägers herausragt.

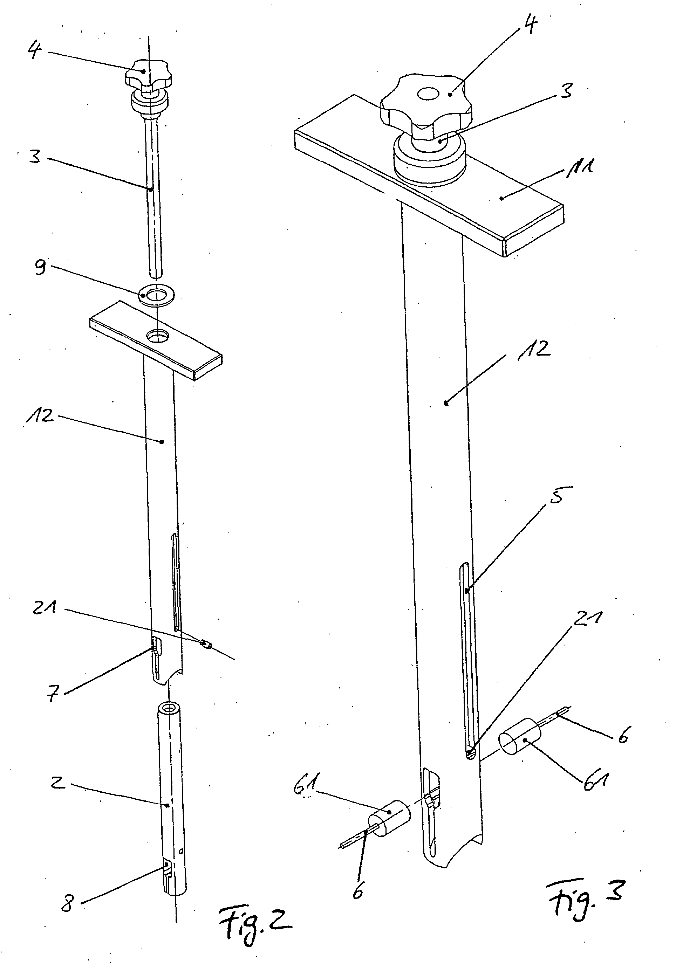

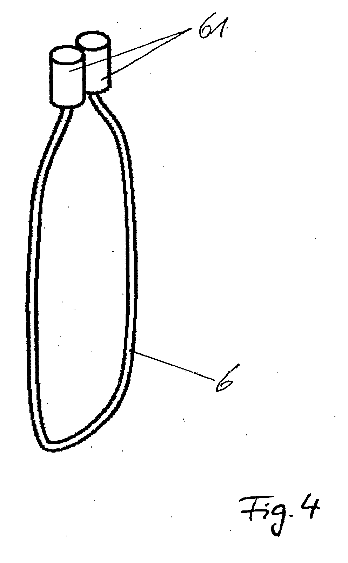

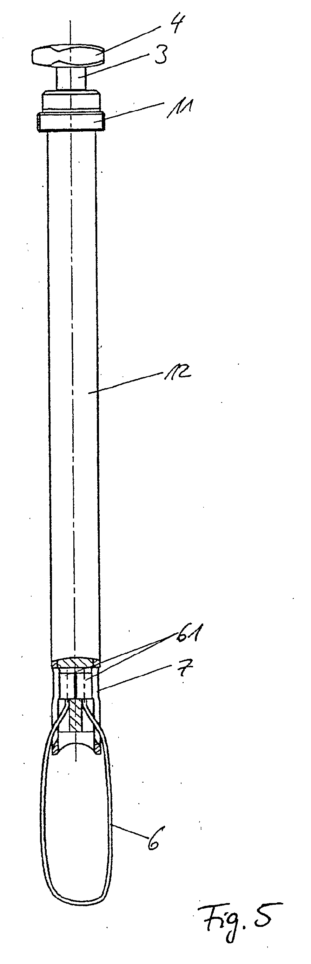

Die Schlaufe 6 besitzt, wie in Fig. 4 dargestellt, zwei freie Enden, die mit Halteelementen 61 versehen sind und der Schenkel 12 weist, wie in Fig. 3 und Fig. 5 dargestellt, ein Langlöch 5 zur Führung der Schraube 21 sowie Ausnehmungen 7 auf, denen räumlich korrespondierend Ausnehmungen 8 in dem Führungsbolzen 2 zugeordnet sind, wobei durch die Ausnehmungen 7 und 8 die Halteelemente 61 einhängbar und durch die Ausnehmungen 8 kraftschlüssig halterbar sind.

Die Schlaufe 6 ist durch ihre Einhängbarkeit vermittels der Halteelemente 61 und der Ausnehmungen 7 und 8 austauschbar, so dass verschieden große Schlaufen einhängbar sind.

Die stabile orthograde Verankerung des erfindungsgemäßen Universalausschlägers erfolgt bei einer bestimmungsgemäßen Verwendung am Halssegment des Prothesenschaftes durch die Schlaufe 6, die besonders vorteilhaft aus geflochtenem, metallischen Material besteht, welche erfindungsgemäß in Form einer Achtertour um die Prothese gelegt wird. Durch Spannung der Schlaufe 6 stützt sich der Universalausschläger am Halssegment des Prothesenschaftes ab. Durch die Form der Kontaktfläche des Ausschlägers erfolgt dessen Selbstzentrierung. Nun kann der "vorgelockerte" Prothesenschaft durch Schläge eines Metallhammers gegen die Ausschlagplatte 11 des T-Stückes 1 aus dem knöchernen Lager gezogen werden.

Der erfindungsgemäße Universalausschläger gestattet durch seine in ihrer Länge einstellbare und mechanisch stabile Anschlingung eine Explantation eines breiten Spektrums von weltweit angebotenen Hüftendoprothesen.

Darüber hinaus ist die Schlaufe als Verschleißteil (bspw. Zerreißen durch Krafteinwirkung bei der Verwendung) einfach und schnell durch eine intakte neue Schlaufe ersetzbar.

Alle in der Beschreibung, den nachfolgenden Ansprüchen und in den Zeichnungen dargestellten Merkmale können sowohl einzeln, als auch in beliebiger Kombination miteinander erfindungswesentlich sein.

### Bezugszeichenliste

- 1: - T-Stück
- 11: - Platte
- 12: - Schenkel
- 2: - Führungsbolzen
- 21: - Schraube
- 3: - Schraubbolzen
- 4: - Kurbel oder Sterrigriff
- 5: - Langloch
- 6: - Schlaufe
- 61: - Halteelemente
- 7: - Ausnelunungen im Schenkel
- 8: - Ausnehmungen im Führungsbolzen
- 9: - Plastik-Ring

## Patentansprüche

1. Universalausschläger für Hüftendoprothesen umfassend
• ein T-Stück (1) bestehend aus einer Platte (11) und einem Schenkel (12), wobei der Schenkel (12) aus einem axialen Rohr gebildet ist,
• einen Führungsbolzen (2) mit Schraube (21),
• einen Schraubbolzen (3) mit einer Kurbel oder einem Sterngriff (4),
wobei der Schraubbolzen (3) drehbar in den Führungsbolzen (12) greift, der Führungsbolzen (2) im Schenkel (12) des T-Stückes (1) axial gelagert und dadurch axial bewegbar ist, und der Führungsbolzen (2) an seinem distalen Ende mit einer Schlaufe (6) verbunden ist, die an dem der Platte (11) gegenüberliegenden Ende des Universalausschlägers aus dem Rohr herausragt, **dadurch gekennzeichnet, dass** die Schlaufe (6) zwei freie Enden besitzt, die mit Halteelementen (61) versehen sind,
der Schenkel (12) ein Langloch (5) zur Führung der Schraube (21) sowie Ausnehmungen (7) aufweist, denen räumlich korrespondierend Ausnehmungen (8) in dem Führungsbolzen (2) zugeordnet sind, wobei durch die Ausnehmungen (7 und 8) die Halteelemente (61) einhängbar und durch die Ausnehmungen (8) kraftschlüssig halterbar sind,
die Schraube (21) radial und gleitend in dem Langloch (5) angeordnet ist, so dass der Freiheitsgrad der Rotation des Führungsbolzens (2) im axialen Schenkel (12) aufhebbar ist und sich der Schraubbolzen (3) auf der Platte (11) des T-Stücks (1) abstützt.

2. Universalausschläger gemäß Anspruch 1, **dadurch gekennzeichnet, dass** die axiale Verschiebung des Führungsbolzens (2) vermittels des axial angeordneten Schraubbolzens (3) erzeugbar ist.

3. Universalausschläger gemäß Anspruch 2, **dadurch gekennzeichnet, dass** der untere Teil des Schraubbolzens (3) durch ein Gewinde mit dem Führungsbolzen (2) verbunden ist, und wobei das Schraubengetriebe im betriebenen Zustand eine axial auf den Führungsbolzen (2) wirkende Zugkraft bewirkt, so dass dieser in das Rohr des T-Stücks (1) gezogen und dabei die Schlaufe (6) in ihrem eingehängten Zustand bewegt wird.

4. Universalausschläger gemäß Anspruch 1, **dadurch gekennzeichnet, dass** die Schlaufe (6) aus geflochtenem metallischen Material besteht.

5. Universalausschläger gemäß Anspruch 4, **dadurch gekennzeichnet, dass** die Schlaufe (6) eine Drahtschlaufe ist.

6. Universalausschläger gemäß einem oder mehrerer der voran stehenden Ansprüche, **dadurch gekennzeichnet, dass** die Schlaufe (6) in Form einer Achtertour um eine Prothese legbar ist.

## Claims

1. Universal extractor for hip endoprostheses comprising:
• one T-piece (1) consisting of a plate (11) and a limb (12) which is formed from an axial tube,
• a guide bolt (2) with screw (21),
• a screw bolt (3) with a crank or star handle (4),
and the screw bolt (3) rotatably meshes with the guide bolt (2), the guide bolt (2) is axially supported in the limb (12) of the T-piece (1) and thus it is axially movable, and the distal end of the guide bolt (2) is connected with a loop (6) protruding from the tube at the end of the universal extractor that is positioned opposite to the plate (11), wherein the loop (6) has two free ends that are provided with retaining elements (61),
the limb (12) has both an elongated hole (5) for guiding the screw (21) and recesses (7) to which spatially corresponding recesses (8) in the guide bolt (2) are assigned, and the retaining elements (61) can be suspended through the recesses (7 and 8) and be supported by the recesses (8) in a force-fit manner,
the screw (21) is arranged radially and glidingly in the elongated hole (5) so that the degree of freedom of the rotation of the guide bolt (2) in the axial limb (12) can be overridden and the screw bolt (3) supports itself on the plate (11) of the T-piece (1).

2. Universal extractor according to claim 1, wherein the guide bolt (2) can be axially shifted by means of the axially arranged screw bolt (3).

3. Universal extractor according to claim 2, wherein the lower part of the screw bolt (3) is connected with the guide bolt (2) via a thread and in operating mode the screw-type-gearing applies an axial tractive force on the guide bolt (2) so that the guide bolt (2) is pulled into the tube of the T-piece (1) and thus the loop (6) is moved in its suspended position.

4. Universal extractor according to claim 1, wherein the loop (6) consists of braided metallic material.

5. Universal extractor according to claim 4, wherein the loop (6) is a wire loop.

6. Universal extractor according to one or several of the preceding claims, wherein the loop (6) can be laid in form of an eight around a prosthesis.

## Revendications

1. Extracteur universel pour prothèses totales de l'articulation de la hanche contenant
• une pièce en T(1) qui se compose d'une plaque (11) et d'une branche (12) étant formée d'un tube axial,
• une broche guide (2) avec vis (21),
• un boulon fileté (3) avec une manivelle ou une poignée-étoile (4), le boulon fileté (3) engrenant de façon tournante dans la broche guide (2), la broche guide (2) étant supportée axialement dans la branche (12) de la pièce en T (1) et, de cette façon, étant mouvable axialement, et l'extrémité distale de la broche guide (2) étant liée avec un noeud coulant (6), qui sort du tube sur l'extrémité opposée à la plaque (11) de l'extracteur universel, est **caractérisé en ce que** le noeud coulant (6) possède deux extrémités libres, qui sont munies des éléments de support (61),
la branche (12) est munie d'un trou oblong (5) pour guider la vis (21) ainsi que des creux (7), auxquels sont assignés des creux (8) dans la broche guide (2) de façon correspondante, et grâce aux creux (7 et 8) les éléments de support (61) peuvent être accrochés et retenus par friction au moyen des creux (8),
la vis (21) est disposée radialement et en coulissant dans le trou oblong (5) de sorte que le degré de liberté de rotation de la broche guide (2) dans la branche (12) axiale puisse être supprimé et
le boulon fileté (3) s'appuie sur la plaque (11) de la pièce en T (1).

2. Extracteur universel suivant la revendication 1 est **caractérisé en ce que** le déplacement axial de la broche guide (2) peut être provoqué au moyen du boulon fileté (3) disposé axialement.

3. Extracteur universel suivant la revendication 2 est **caractérisé en ce que** la partie inférieure du boulon fileté (3) est liée à la broche guide (2) grâce à un filetage, et le mouvement à vis en état activé provoque une traction agissant axialement sur la broche guide (2) de sorte que la broche guide (2) soit introduite dans le tube de la pièce en T (1) en mouvant le noeud coulant (6) en son état accroché.

4. Extracteur universel suivant la revendication 1 est **caractérisé en ce que** le noeud coulant (6) se compose d'un matériau métallique tressé.

5. Extracteur universel suivant la revendication 4 est **caractérisé en ce que** le noeud coulant (6) est une boucle en fil métallique.

6. Extracteur universel suivant une ou plusieurs des revendications précédentes est **caractérisée en ce que** le noeud coulant (6) peut être placé autour d'une prothèse en formant des huits.
